# EUROPEAN PATENT SPECIFICATION

(11) **EP 0 866 861 B1**
(45) Date of publication and mention of the grant of the patent: **25.02.2004**
(21) Application number: 96937801.7
(22) Date of filing: 01.11.1996
(51) Int. Cl.: C12N 15/16, C07K 14/64, C12N 5/10, C12N 1/21, C12N 1/19, C07K 16/26, A61K 38/22, A61K 39/395, C07K 19/00

(54) **MOLECULAR CLONING AND CHARACTERIZATION OF MOLECULES RELATED TO RELAXIN AND THE INSULIN FAMILY OF LIGANDS**
MOLEKULARE KLONIERUNG UND CHARAKTERISIERUNG VON MOLEKÜLEN, WELCHE VERWANDT SIND MIT RELAXIN UND LIGANDEN DER INSULINFAMILIE
CLONAGE MOLECULAIRE ET CARACTERISATION DE MOLECULES LIEES A LA RELAXINE ET A LA FAMILLE INSULINIQUE DE LIGANDS

(30) Priority: 03.11.1995 US 6221 P; 21.02.1996 US 12016 P
(43) Date of publication of application: 30.09.1998
(73) Proprietor: REGENERON PHARMACEUTICALS, INC., Tarrytown, NY 10591-6707 (US)
(72) Inventor: DAVIS, Samuel, New York, NY 10024 (US)
(74) Representative: Duckworth, Timothy John
(86) International application number: PCT/US1996/017342
(87) International publication number: WO 1997/016549

(56) References cited:
- GENOMICS 29 (2). 1995. 465-470. ISSN: 0888-7543, 20 September 1995, XP000644716 CHASSIN D ET AL: "Cloning of a new member of the insulin gene superfamily (INSL4) expressed in human placenta."
- NATURE, vol. 301, 1983, pages 628-631, XP002025421 P. HUDSON ET AL: "Structure of a genomic clone encoding biologically active human relaxin"

## Description

This invention concerns the molecular cloning and characterization of nucleic acid sequences that encode molecules, hereinafter referred to as relaxin-related factors, that are related to the hormone relaxin and to the insulin family of ligands. More specifically, this invention provides isolated nucleic acid molecules encoding relaxin-related factors. The nucleic acid molecules may be expressed in suitable host cells and are thus useful for the production of the relaxin-related factors and relaxin-related factor ligandbodies, as well as for the production of probes that may be used to further screen for additional related family members. The invention further provides isolated relaxin-related factors substantially free of other proteins. The isolated relaxin-related factor proteins, as well as derivatives thereof, may be used for the preparation of antibodies and therapeutic compositions and as growth factors for maintaining cultures of cells which have receptors for relaxin-related factor.

### BACKGROUND OF THE INVENTION

Relaxin is a polypeptide hormone that has been found in the female of all species studied. Relaxin is synthesized and stored in the corpora lutea of ovaries during pregnancy and is released into the blood stream prior to parturition. The corpus luteum of pregnancy is the main source of relaxin in many species but in others the decidua is apparently of greater importance. Hisaw suggested an important role for relaxin in mammals through its effects in dilating the pubic symphysis, thus facilitating the birth process. (Hisaw, F. L., Proc. Soc. Exp. Biol. Med. 23, 661-663 (1926)). Relaxin has since been found to have many other possible roles, including preparation of the endometrium for implantation, inhibition of uterine activity in early pregnancy, remodeling of the uterine stroma during pregnancy, cervical ripening and the initiation of parturition. Relaxin's main cellular action in pregnancy may be to drive collagen biosynthesis in its target organs, thus facilitating the remodeling of the connective tissue. Published PCT application W08907945 published September 8, 1989 filed by Genentech, Inc., discloses relaxin compositions which are stated to be useful for modulating the reproductive physiology of mammals during pregnancy and parturition. Relaxin has been reported to be capable of altering the nature of connective tissue, influencing smooth muscle contraction, and to have the general properties of a growth factor. It has also been reported that relaxin may influence intraocular pressure when administered in pharmacologic doses. (Kass, M.A. et al., Survey of Opththalmology 22(3): 153-176 (1977)).

Although the corpora lutea of the ovary as well as decidual and placental tissues are the most likely sites for expression of relaxin-related genes, relaxin has also been found in other tissues such as prostatic fluid and testis. There is evidence in pigs for relaxin gene expression and translation into protein in the theca interna cells of the preovulatory follicle, the corpus luteum of the cycle and the uterus. Relaxin has been identified in boar seminal plasma and can maintain or increase sperm motility. (See Bagnell, C.A., et al., J. Reprod. & Fert. (Supp.) 48:127-138 (1993) for review). It has been reported that specific, high activity human relaxin binding sites are present in discrete regions of the rat brain such that the distribution of some of these sites may be consistent with a role for relaxin in control of vascular volume and blood pressure. (Osheroff, P.L. and Phillips, H.S., Proc. Nat. Acad. Sci. (USA) 88 (15): 6413-6417 (1991)).

Biologically active relaxin consists of two peptide chains (known as the A and B chains) held together by one intra-chain and two inter-chain disulfide bonds. The structure thus closely resembles insulin, which has led to speculation of a common ancestral gene for these hormones (Schwabe, C., et al., Biochem. Biophys. Res. Commun. 75,503-510 (1977); James, R., *et al.,* Nature, 267:544-546 (1977)). Relaxin is considered a member of the insulin family, which also includes insulin, IGF1, IGF2 and Leydig insulin-like protein. cDNA clones for both rat and porcine relaxins have been obtained via recombinant DNA techniques. (Hudson, P., Haley, J., Cronk, M., Shine, J. and Niall, H. Nature, 291:127-131 (1981)). Further, the molecular cloning and characterisation of a gene sequence coding for human relaxin has been described. (U.S. Patent No. 4,871,460 issued October 3, 1989 to Hudson, *et al.*).

### SUMMARY OF THE INVENTION

This invention concerns the molecular cloning and characterisation of nucleic acids encoding molecules, referred to as relaxin-related factors, that are related to the hormone relaxin and to the insulin family of ligands.

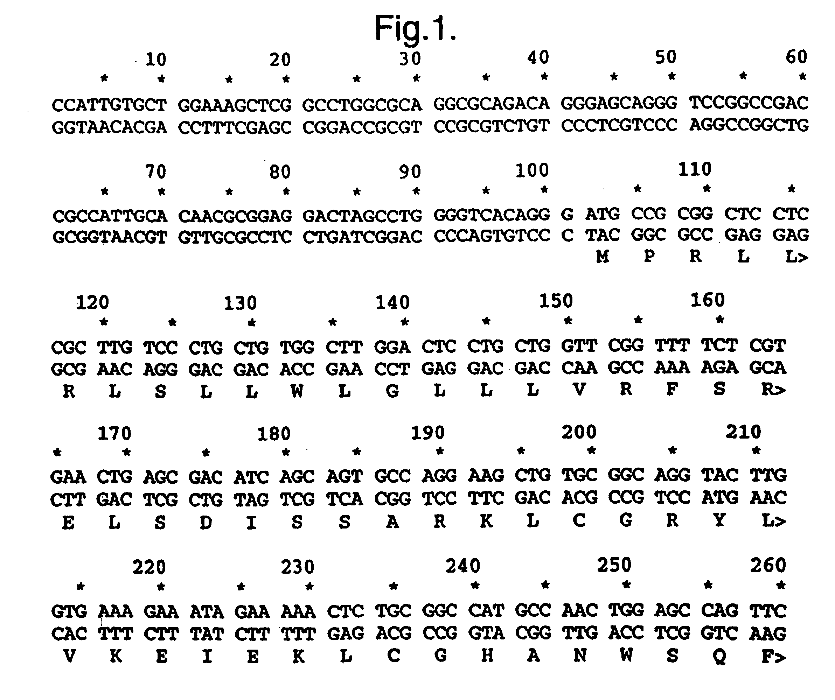

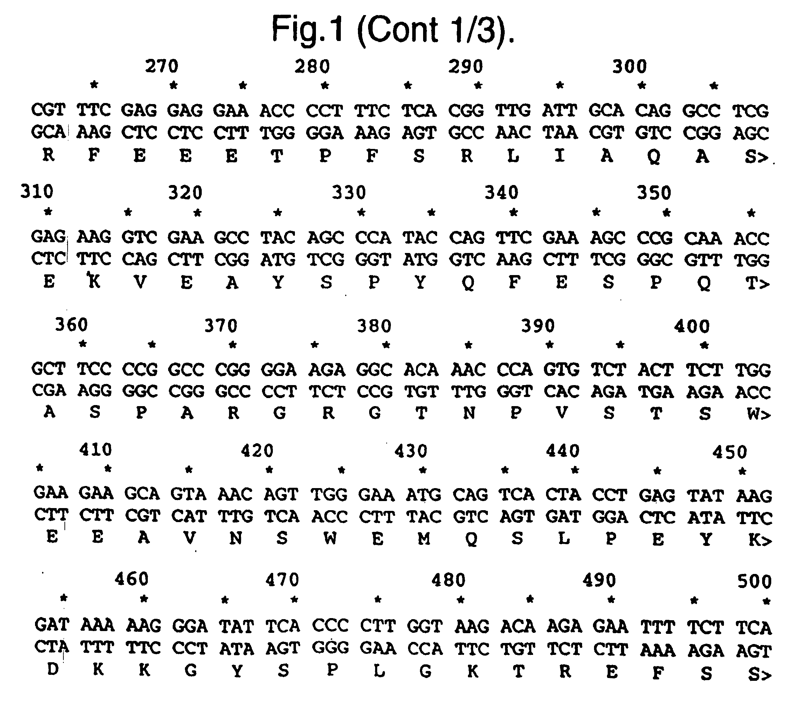

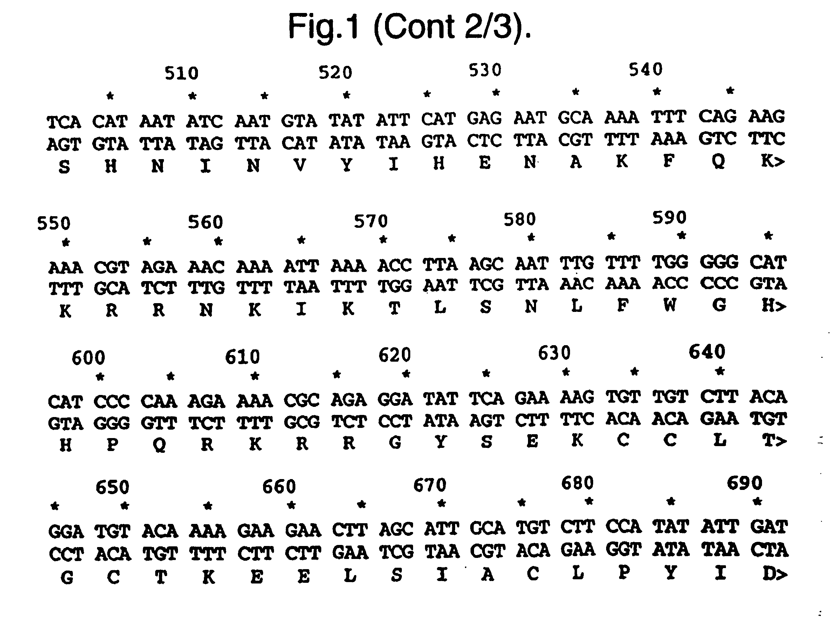

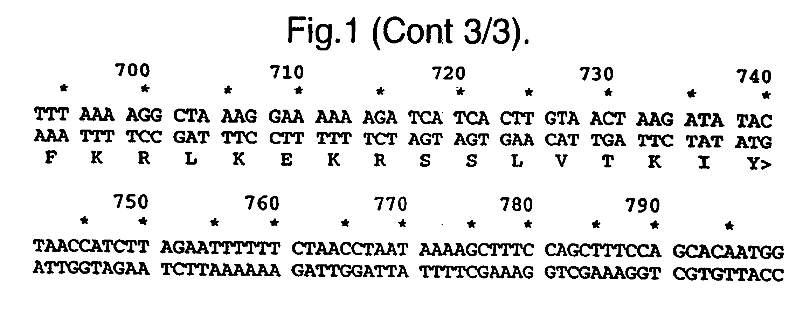

More specifically, this invention provides an isolated nucleic acid molecule encoding relaxin-related factor-1 (RRF-1). The present invention thus provides an isolated nucleic acid molecule which encodes the amino acid sequence of the relaxin related factor as set forth in Figure 1. The invention also provides vectors which comprise such a nucleic acid as well as host cells which are genetically engineered to produce RRF-1.

The present invention further provides a relaxin-related factor as set forth in Figure 1 substantially free of other proteins. The invention further provides for an antibody which specifically binds RRF-1. The antibodies may be polyclonal or monoclonal.

The invention also provides for a pharmaceutical composition comprising RRF-1 and a pharmaceutically acceptable carrier. The invention further provides for a pharmaceutical composition comprising an antibody which specifically binds RRF-1 and a pharmaceutically acceptable carrier.

The invention further provides for a relaxin-related factor ligandbody which comprises RRF-1 fused to an immunoglobulin constant region.

The invention further provides RRF-1, an antibody specific to RRF-1, a pharmaceutical composition comprising RRF-1 or any antibody specific to RRF-1 or an RRF-1 ligandbody for use in a method of treatment of the human or animal body or in a method of diagnosis.

### BRIEF DESCRIPTION OF THE FIGURES

FIGURE 1 - Nucleotide and deduced amino acid (single letter code) sequences of relaxin-related factor-1 (RRF-1)

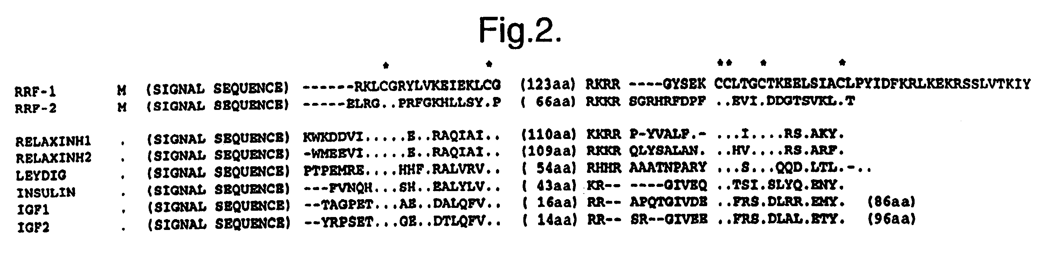

FIGURE 2 - Comparative alignment of amino acid sequences of relaxin-related factor-1 (RRF-1) and relaxin-related factor-2 (RRF-2) with amino acid sequences of other members of the insulin family including: relaxin H1; relaxin H2; Leydig insulin-like protein (leydig); insulin; insulin-like growth factor 1 (IGF1); and insulin-like growth factor 2 (IGF2). Conserved cysteine residues are indicated by asterisks above the RRF-1 sequence. Dots indicate identical amino acid residues. Dashes represent artificial insertions for purposes of alignment. Deletions ranging from 14-123 amino acids as well as deletions of the signal sequences have been made for purposes of alignment and are indicated in parentheses.

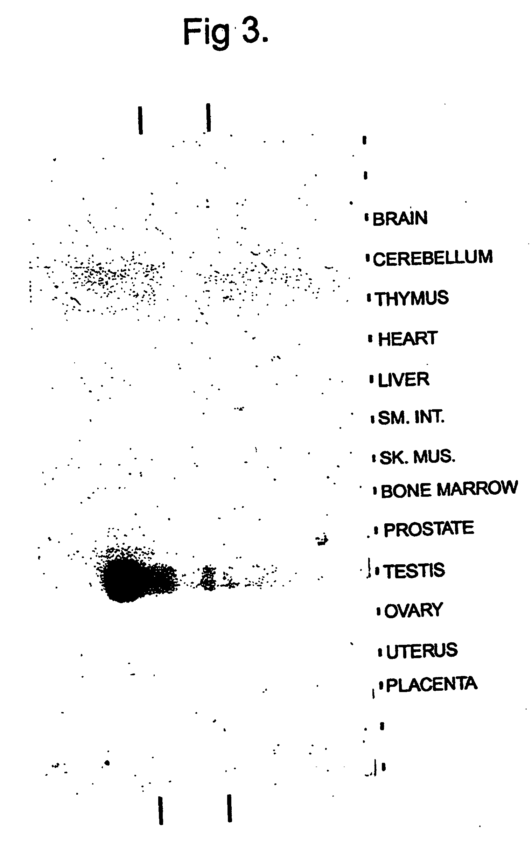

FIGURE 3 - Northern blot showing expression of message encoding relaxin-related factor-1 (RRF-1) in human testis, but not in any other human tissue tested. Lanes, from left, are Brain, Cerebellum, Thymus, Heart, Liver, Small Intestine, Skeletal Muscle, Bone Marrow, Prostate, Testis, Ovary; Uterus, Placenta.

### DETAILED DESCRIPTION OF THE INVENTION

This invention concerns the molecular cloning and characterization of nucleic acids encoding molecules, hereinafter referred to as relaxin-related factors, that are related to the hormone relaxin and to the insulin family of ligands. More specifically, this invention provides an isolated nucleic acid molecule encoding relaxin-related factor-1 (RRF-1). The present invention also provides a nucleotide sequence that encodes RRF-1 as well as cells which are genetically engineered to produce the molecule, by e.g. transfection, transduction, infection, electroporation, or microinjection of a nucleic acid encoding RRF-1 as described herein in a suitable expression vector.

The invention also provides for a vector which comprises an isolated nucleic acid molecule encoding relaxin-related factor-1. In one embodiment, the vector is operatively linked to an expression control sequence capable of directing its expression in a host cell. In another embodiment, the vector is a plasmid.

The invention further provides for a host-vector system for the production of a relaxin-related factor, polypeptide, or peptide fragment thereof which comprises a vector of the invention in a host cell. The host cell preferably may be a bacterial cell such as E. coli. or a yeast, insect or mammalian cell. The invention further provides for a method of producing a relaxin-related factor which comprises growing cells of a host-vector system under conditions permitting production of the relaxin-related factor, and recovering the relaxin-related factor so produced. In one embodiment of the invention the relaxin-related factor is RRF-1.

The invention further provides for an isolated relaxin-related factor RRF-1 substantially free of other proteins. In one embodiment, the isolated relaxin-related factor is encoded by the nucleotide sequence comprising the coding region of RRF-1 as set forth in Figure 1.

The invention further provides for an antibody which specifically binds relaxin-related factor RRF-1. The antibody may be a polyclonal or a monoclonal antibody.

The invention further provides for a pharmaceutical composition comprising relaxin-related factor RRF-1 and a pharmaceutically acceptable carrier. The invention further provides for a pharmaceutical composition comprising an antibody which specifically binds relaxin-related factor RRF-1 and a pharmaceutically acceptable carrier.

The invention further provides for a relaxin-related factor ligandbody which comprises relaxin-related factorRRF-1 fused to an immunoglobulin constant region. In one embodiment of the relaxin-related factor ligandbody, the immunoglobulin constant region is the Fc portion of human IgG1.

As used herein, the term "relaxin-related factor" includes the RRF-1 molecules described herein, as well as functionally equivalent molecules in which amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Also included within the scope of the term "relaxin-related factor" are fragments or derivatives thereof which exhibit the same or similar biological activity and derivatives which are differentially modified during or after translation, e.g. by glycosylation, proteolytic cleavage, linkage to an antibody molecule or other cellular ligand, etc.

One skilled in the art will also recognize that DNA and RNA sequences may hybridize to a relaxin-related factor encoding sequence, under conditions of moderate stringency, as defined in, for example, Sambrook, et al. Molecular Cloning: A Laboratory Manual, 2 ed. Vol. 1, pp. 101-104, Cold Spring Harbor Laboratory Press (1989).

A nucleic acid encoding a relaxin-related factor as described herein will facilitate the genetic engineering of cells to produce the relaxin-related factor by, e.g., transfection, transduction, electroporation, or microinjection of a suitable host cell using a nucleotide sequence encoding the relaxin-related factor in a suitable expression vector, thus forming a host cell vector system for the production of a polypeptide having the biological activity of the relaxin-related factor. A suitable host cell may be selected from the group consisting of bacterial cells (such as E. coli), yeast cells, fungal cells, insect cells and animal cells. Suitable animal cells include, but are not limited to, Vero cells, HeLa cells, Cos cells, CV1 cells and various primary mammalian cells.

Any of the methods known to one skilled in the art for the insertion of DNA fragments into a vector may be used to construct expression vectors encoding the relaxin-related factor using appropriate transcriptional/translational control signals and the protein coding sequences. These methods may include in vitro recombinant DNA and synthetic techniques and in vivo recombinations (genetic recombination). Expression of a nucleotide sequence encoding relaxin-related factor or peptide fragments thereof may be regulated by a second nucleotide sequence so that the protein or peptide is expressed in a host transformed with the recombinant DNA molecule. For example, expression of the relaxin-related factor described herein may be controlled by any promoter/enhancer element known in the art. Promoters which may be used to control expression of the relaxin-related factor include, but are not limited to the long terminal repeat as described in Squinto et al., (Cell 65:1-20 (1991)); the SV40 early promoter region (Bemoist and Chambon, Nature 290:304-310), the CMV promoter, the M-MuLV 5' terminal repeat, the promoter contained in the 3' long terminal repeat of Rous sarcoma virus (Yamamoto, et al., Cell 22:787-797 (1980)), the herpes thymidine kinase promoter (Wagner et al., Proc. Natl. Acad. Sci. U.S.A. 78:144-1445 (1981)), the adenovirus promoter, the regulatory sequences of the metallothioein gene (Brinster et al., Nature 296:39-42 (1982)); prokaryotic expression vectors such as the β-lactamase promoter (Villa-Kamaroff, et al., Proc. Natl. Acad. Sci. U.S.A. 75:3727-3731 (1978)), or the tac promoter (DeBoer, et al., Proc. Natl. Acad. Sci. U.S.A. 80:21-25 (1983)), see also "Useful proteins from recombinant bacteria" in Scientific American, 242:74-94 (1980); promoter elements from yeast or other fungi such as the Gal 4 promoter, the ADH (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., Cell 38:639-646 (1984); Ornitz et al., Cold Spring Harbor Symp. Quant Biol. 50:399-409 (1986); MacDonald, Hepatology 7:425-515 (1987); insulin gene control region which is active in pancreatic beta cells (Hanahan, Nature 315:115-122 (1985)), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94); myelin basic protein gene control region which is active in oligodendrocyte cells in the brain (Readhead et al., 1987, Cell 48:703-712); myosin light chain-2 gene control region which is active in skeletal muscle (Shani, 1985, Nature 314:283-286), and gonadotropic releasing hormone gene control region which is active in the hypothalamus (Mason et al., 1986, Science 234:1372-1378). The invention further provides for the production of antisense compounds which are capable of specifically hybridizing with a sequence of RNA encoding the relaxin-related factor to modulate its expression. (Ecker, U.S. Patent No. 5,166,195, issued November 24, 1992).

Thus, according to the invention, expression vectors capable of being replicated in a bacterial or eukaryotic host comprising a nucleic acid encoding a relaxin-related factor are used to transfect a host and thereby direct expression of such nucleic acid to produce the relaxin-related factor, which may then be recovered in a biologically active form. As used herein, a biologically active form includes a form capable of binding to a receptor for the relaxin-related factor and causing a biological response such as a differentiated function or influencing the phenotype of the cell expressing the receptor.

Expression vectors containing the gene inserts can be identified by four general approaches: (a) DNA-DNA hybridization, (b) presence or absence of "marker" gene functions, (c) expression of inserted sequences and (d) PCR detection. In the first approach, the presence of a foreign gene inserted in an expression vector can be detected by DNA-DNA hybridization using probes comprising sequences that are homologous to an inserted relaxin-related factor encoding gene. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign genes in the vector. For example, if a nucleic acid encoding relaxin-related factor is inserted within the marker gene sequence of the vector, recombinants containing the insert can be identified by the absence of the marker gene function. In the third approach, recombinant expression vectors can be identified by assaying the foreign gene product expressed by the recombinant. Such assays can be based, for example, on the physical or functional properties of a relaxin-related factor gene product, for example, by binding of the relaxin-related factor to its receptor or portion thereof which may be tagged with, for example, a detectable antibody or portion thereof or by binding to antibodies produced against the relaxin-related factor or a portion thereof. Cells of the present invention may transiently, or preferably constitutively and permanently, express relaxin-related factor as described herein. In the fourth approach, DNA nucleotide primers can be prepared corresponding to a relaxin-related factor specific DNA sequence. These primers could then be used to PCR a relaxin-related factor gene fragment. (PCR Protocols: A Guide To Methods and Applications, Edited by Michael A. Innis et al., Academic Press (1990)).

A recombinant relaxin-related factor may be purified by any technique which allows for the subsequent formation of a stable, biologically active protein. For example, and not by way of limitation, a relaxin-related factor may be recovered from cells either as soluble proteins or as inclusion bodies, from which they may be extracted quantitatively by 8M guanidinium hydrochloride and dialysis. In order to further purify the relaxin-related factor, conventional ion exchange chromatography, hydrophobic interaction chromatography, reverse phase chromatography or gel filtration may be used.

In additional embodiments of the invention, a recombinant relaxin-related factor encoding gene may be used to inactivate or "knock out" the endogenous gene by homologous recombination, and thereby create a relaxin-related factor deficient cell, tissue, or animal. For example, and not by way of limitation, the recombinant relaxin-related factor encoding gene may be engineered to contain an insertional mutation, for example the neo gene, which would inactivate the native relaxin-related factor encoding gene. Such a construct, under the control of a suitable promoter, may be introduced into a cell, such as an embryonic stem cell, by a technique such as transfection, transduction, or injection. Cells containing the construct may then be selected by G418 resistance. Cells which lack an intact relaxin-related factor encoding gene may then be identified, e.g. by Southern blotting, PCR detection, Northern blotting or assay of expression. Cells lacking an intact relaxin-related factor encoding gene may then be fused to early embryo cells to generate transgenic animals deficient in such relaxin-related factor. Such an animal may be used to define specific in vivo processes, normally dependent upon the relaxin-related factor.

The present invention also provides for antibodies to a relaxin-related factor described herein which are useful for detection of the factor in, for example, diagnostic applications. For the preparation of monoclonal antibodies directed toward a relaxin-related factor, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in "Monoclonal Antibodies and Cancer Therapy," Alan R Liss, Inc. pp. 77-96) and the like are within the scope of the present invention.

Monoclonal antibodies for diagnostic or therapeutic use may be human monoclonal antibodies or chimeric human-mouse (or other species) monoclonal antibodies. Human monoclonal antibodies may be made by any of numerous techniques known in the art (e.g., Teng et al., 1983, Proc. Natl. Acad. Sci. U.S.A. 80:7308-7312; Kozbor et al., 1983, Immunology Today 4:72-79; Olsson et al., 1982, Meth. Enzymol. 92:3-16). Chimeric antibody molecules may be prepared containing a mouse antigen-binding domain with human constant regions (Morrison et al., 1984, Proc. Natl. Acad. Sci. U.S.A. 81:6851, Takeda et al., 1985, Nature 314:452).

Various procedures known in the art may be used for the production of polyclonal antibodies to epitopes of a relaxin-related factor described herein. For the production of antibody, various host animals can be immunized by injection with a relaxin-related factor, or a peptide fragment or derivative thereof, including but not limited to rabbits, mice and rats. Various adjuvants may be used to increase the immunological response, depending on the host species and including, but not limited to, Freund's adjuvant (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

A molecular clone of an antibody to a selected relaxin-related factor epitope can be prepared by known techniques. Recombinant DNA methodology (see e.g., Maniatis et al., 1982, Molecular Cloning, A Laboratory Manual, Cold Spring Harbor Laboratory, Cold Spring Harbor, New York) may be used to construct nucleotide sequences which encode a monoclonal antibody molecule, or antigen binding region thereof.

The present invention provides for antibody molecules as well as fragments of such antibody molecules. Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment; and the Fab fragments which can be generated by treating the antibody molecule with papain and a reducing agent. Antibody molecules may be purified by known techniques, e.g. immunoabsorption or immunoaffinity chromatography, chromatographic methods such as HPLC (high performance liquid chromatography), or a combination thereof.

Having isolated a nucleic acid molecule encoding a relaxin-related factor, it should pose no great difficulty to create an expression construct that would yield a secreted protein consisting of the entire coding sequence of the relaxin-related factor fused to the human immunoglobulin gamma-1 constant region (IgG1 Fc). These fusion proteins are called "relaxin-related factor ligandbodies." The relaxin-related factor may be RRF-1.

The Fc portion may be prepared as follows. A DNA fragment encoding the Fc portion of human IgG1 that spans from the hinge region to the carboxy-terminus of the protein may be amplified from human placental cDNA by PCR with oligonucleotides corresponding to the published sequence of human IgG1; the resulting DNA fragment may be cloned into a plasmid vector. Appropriate DNA restriction fragments from a plasmid encoding the full-length relaxin-related factor and from the human IgG1 Fc plasmid may be ligated on either side of a short PCR-derived fragment that is designed so as to fuse, in-frame, the chosen relaxin-related factor with human IgG1 Fc protein-coding sequences.

The present invention therefore also provides for a relaxin-related factor ligandbody which specifically binds the chosen relaxin-related factor's receptor. In one embodiment, the present invention provides for a relaxin-related factor ligandbody which comprises a relaxin-related factor ligand fused to an immunoglobulin constant region. In a further embodiment, the ligandbody comprises a relaxin-related factor and the Fc portion of human IgG1. In a further embodiment, the ligandbody comprises RRF-1 and the Fc portion of human IgG1. A relaxin-related factor ligandbody as described above may be used in a method of treatment of the human or animal body, or in a method of diagnosis.

By way of nonlimiting example, relaxin-related factor ligandbodies may be useful for the delivery of toxins to a receptor bearing cell. Alternatively, other molecules, such as growth factors, cytokines or nutrients, may be delivered to a relaxin-related factor receptor bearing cell via relaxin-related factor ligandbodies. A relaxin-related factor ligandbody could also be used as a diagnostic reagent for a relaxin-related factor receptor, to detect a receptor in vivo or in vitro. A relaxin-related factor ligandbody may be useful as a diagnostic reagent for detecting a relaxin-related factor receptor by, for example, tissue staining or whole body imaging.

The present invention also provides for pharmaceutical compositions comprising a relaxin-related factor ligand or ligandbody described herein, peptide fragments thereof, or derivatives in a pharmacologically acceptable vehicle. The relaxin-related factor protein, or peptide fragments or derivatives thereof, may be administered systemically or locally. Any appropriate mode of administration known in the art may be used including, but not limited to, intravenous, intrathecal, intraarterial, intranasal, oral, subcutaneous, intraperitoneal, or by local injection or surgical implant. Sustained release formulations are also provided for.

This invention will be better understood from the Examples which follow. However, one skilled in the art will readily appreciate that the specific methods and results discussed are not limiting but are merely illustrative of the invention.

### EXAMPLE 1 - CLONING OF A MOLECULE RELATED TO RELAXIN AND THE INSULIN FAMILY OF LIGANDS

A search of the database of expressed sequence tags using the sequence of relaxin (a member of the insulin family, which also includes insulin, IGF1, IGF2, and "Leydig insulin-like protein") as the test sequence revealed the existence of a sequence, found in human testis, related to relaxin at the amino acid level. The hallmarks of the insulin family of proteins consist mainly of a particular arrangement of cysteine residues, two near the amino terminus and four towards the carboxy terminus, which form a characteristic structure of disulfide bonds. The sequence found on the database contained the first two cysteines, along with a few other residues in their immediate vicinity that are also characteristic of the insulin family. This homology was rather weak, and the presence of a stop codon in the middle of the database sequence, with no evidence for the characteristic C-terminal cysteines, raised further doubts about whether this sequence represented an authentic new insulin family member.

Oligonucleotides were synthesized corresponding to the database sequence and were assembled by PCR to constitute a 400-base probe. A human testis cDNA library was constructed and then screened with this synthetic probe. Several clones resulting from this screen were sequenced, and it was found that at least one of the clones contained an open reading frame whose predicted protein sequence displayed all the expected features of a new insulin family member, particularly with regard to the cluster of four cysteines at the C-terminus of the molecule. The nucleotide and deduced amino acid (single letter code) sequences of the molecule are shown in Figure 1. Overall, the molecule is more closely related to relaxin than to any of the other insulin family members. The molecule was therefore called relaxin-related factor-1 (RRF-1). Figure 2 shows a comparative alignment of the relaxin-related factor-1 amino acid sequence with the sequences of other members of the insulin family.

Northern blot analysis was performed by running samples of adult human poly A RNA on a 1% agarose-formaldehyde gel, transferring to a nylon membrane and probing with a 400 base pair probe derived from the nucleotide sequence of the relaxin-related factor. As shown in Figure 3, the message for RRF-1 was highly expressed in human testis, but was not detected in any of the other tissue samples that were assayed, including brain, cerebellum, thymus, heart, liver, small intestine, skeletal muscle, bone marrow, prostate, ovary, uterus, and placenta. Because production of RRF-1 appears to be specific to testis, it may be involved in the maturation of sperm and therefore may have a role in treatment of fertility disorders.

### EXAMPLE 2 - EXPRESSION OF HUMAN RELAXIN-RELATED FACTOR-1 cDNA

Expression vectors were constructed containing the human relaxin-related factor-1 cDNA. One version had a triple-myc tag inserted at its C-terminus to make the protein detectable. COS-7 cells were transiently transfected with either the expression vector or a control vector by the DEAE-dextran transfection protocol. Briefly, COS-7 cells were plated at a density of 1.0 x 10⁶ cells per 100 mm plate 24 hours prior to transfection. For transfection, the cells were cultured in serum-free DMEM containing 400 µg/ml of DEAE-dextran, 1 µM chloroquine, and 2 mM glutamine, and 1 µg of the appropriate DNA for 3-4 hours at 37°C in an atmosphere of 5% CO₂. The transfection media was aspirated and replaced with phosphate-buffered saline with 10% DMSO for 2-3 min. Following this DMSO "shock", the COS-7 cells were placed into DMEM with 1% each of penicillin and streptomycin, and 2 mM glutamine. COS media containing secreted ligand was harvested after three days. Expression of the myc-tagged version was verified by Western blot analysis of the supernatants, using antibodies against the myc tag as a probe.

## Claims

1. An isolated nucleic acid molecule which encodes the amino acid sequence of the relaxin related factor as set forth in Figure 1.

2. A nucleic acid according to claim 1 comprising the coding region of the relaxin-related factor as set forth in Figure 1.

3. A vector which comprises a nucleic acid molecule of claim 1 or 2.

4. A vector according to claim 3, wherein the nucleic acid molecule is operatively linked to an expression control sequence capable of directing expression of said nucleic acid in a host cell.

5. A plasmid according to claim 3 or 4.

6. A relaxin-related factor as set forth in Figure 1, substantially free of other proteins.

7. A host-vector system for the production of a relaxin-related factor which comprises a vector of claim 4 in a host cell.

8. A host-vector system according to claim 7, wherein the host cell is a bacterial, yeast, insect or mammalian cell.

9. A method of producing a relaxin-related factor which comprises growing cells of a host-vector system of claim 7 or 8, under conditions permitting production of the relaxin-related factor, and recovering the relaxin-related factor so produced.

10. An antibody which specifically binds the relaxin-related factor of claim 6.

11. An antibody according to claim 10, which is a monoclonal antibody.

12. A pharmaceutical composition comprising a relaxin-related factor according to claim 6 or an antibody according to claim 10 or 11, and a pharmaceutically acceptable carrier.

13. A relaxin-related factor according to claim 6, an antibody according to claim 10 or 11, or a composition according to claim 12, for use in a method of treatment of the human or animal body, or in a method of diagnosis.

14. A ligandbody which comprises a relaxin-related factor as defined in claim 6 fused to an immunoglobulin constant region.

15. A ligandbody according to claim 14, wherein the immunoglobulin constant region is the Fc portion of human IgG1.

16. A ligandbody according to claim 14 or 15, for use in a method of treatment of the human or animal body, or in a method of diagnosis.

## Patentansprüche

1. Isoliertes Nucleinsäuremolekül, welches die Aminosäuresequenz des Relaxin-ähnlichen Faktors codiert wie in Abbildung 1 dargestellt.

2. Nucleinsäure nach Anspruch 1, umfassend den codierenden Bereich des Relaxin-ähnlichen Faktors wie in Abbildung 1 dargestellt.

3. Vektor, umfassend ein Nucleinsäuremolekül nach Anspruch 1 oder 2.

4. Vektor nach Anspruch 3, wobei das Nucleinsäuremolekül funktionell verknüpft ist mit einer Expressionskontrollsequenz, die die Expressionssteuerung der Nucleinsäure in einer Wirtszelle ermöglicht.

5. Plasmid nach Anspruch 3 oder 4.

6. Relaxin-ähnlicher Faktor wie in Anspruch 1 dargestellt, im wesentlichen frei von anderen Proteinen.

7. Wirtsvektorsystem zur Herstellung eines Relaxin-ähnlichen Faktors, welches einen Vektor nach Anspruch 4 in einer Wirtszelle umfaßt.

8. Wirtsvektorsystem nach Anspruch 7, wobei die Wirtszelle eine Bakterien-, Hefe-, Insekten- oder Säugerzelle ist.

9. Methode zur Herstellung eines Relaxin-ähnlichen Faktors, umfassend die Züchtung von Zellen eines Wirtsvektorsystems nach Anspruch 7 oder 8 unter Bedingungen, die die Herstellung des Relaxin-ähnlichen Faktors erlauben, sowie die Gewinnung des so hergestellten Relaxin-ähnlichen Faktors.

10. Antikörper, welcher den Relaxin-ähnlichen Faktor nach Anspruch 6 spezifisch bindet.

11. Antikörper nach Anspruch 10, welcher ein monoclonaler Antikörper ist.

12. Arzneimittel, umfassend einen Relaxin-ähnlichen Faktor nach Anspruch 6 oder einen Antikörper nach Anspruch 10 oder 11 und einen pharmazeutisch verträglichen Träger.

13. Relaxin-ähnlicher Faktor nach Anspruch 6, Antikörper nach Anspruch 10 oder 11 oder Arzneimittel nach Anspruch 12 zur Verwendung in einem Verfahren zur Behandlung des menschlichen oder tierischen Körpers oder in einem Diagnoseverfahren.

14. Ligandenkörper, umfassend einen Relaxin-ähnlichen Faktor nach Anspruch 6, welcher an die konstante Region eines Immunglobulins fusioniert ist.

15. Ligandenkörper nach Anspruch 14, wobei die konstante Region des Immunglobulins der Fc-Bereichs des menschlichen IgG1 ist.

16. Ligandenkörper nach Anspruch 14 oder 15 zur Verwendung in einem Verfahren zur Behandlung des menschlichen oder tierischen Körpers oder in einem Diagnoseverfahren.

## Revendications

1. Molécule isolée d'acide nucléique qui code la séquence d'acides aminés du facteur apparenté à la relaxine selon la figure 1.

2. Acide nucléique selon la revendication 1, comprenant la région codante du facteur apparenté à la relaxine selon la figure 1.

3. Vecteur qui comprend une molécule d'acide nucléique selon la revendication 1 ou 2.

4. Vecteur selon la revendication 3, dans lequel la molécule d'acide nucléique est liée fonctionnellement à une séquence de contrôle de l'expression capable de diriger l'expression dudit acide nucléique dans une cellule hôte.

5. Plasmide selon la revendication 3 ou 4.

6. Facteur apparenté à la relaxine selon la figure 1, essentiellement exempt d'autres protéines.

7. Système hôte-vecteur pour la production d'un facteur apparenté à la relaxine qui comprend un vecteur selon la revendication 4 dans une cellule hôte.

8. Système hôte-vecteur selon la revendication 7, dans lequel la cellule hôte est une cellule de bactérie, de levure, d'insecte ou de mammifère.

9. Procédé de production d'un facteur apparenté à la relaxine qui comprend la culture de cellules d'un système hôte-vecteur selon la revendication 7 ou 8, dans des conditions permettant la production du facteur apparenté à la relaxine, et la récupération du facteur apparenté à la relaxine ainsi produit.

10. Anticorps qui lie spécifiquement le facteur apparenté à la relaxine selon la revendication 6.

11. Anticorps selon la revendication 10, qui est un anticorps monoclonal.

12. Composition pharmaceutique comprenant un facteur apparenté à la relaxine selon la revendication 6 ou un anticorps selon la revendication 10 ou 11, et un support pharmaceutiquement acceptable.

13. Facteur apparenté à la relaxine selon la revendication 6, un anticorps selon la revendication 10 ou 11, ou une composition selon la revendication 12, pour l'usage dans un procédé de traitement du corps humain ou animal, ou dans un procédé de diagnostic.

14. Corps ligand qui comprend un facteur apparenté à la relaxine selon la revendication 6, fusionné avec une région constante d'immunoglobuline.

15. Corps ligand selon la revendication 14, dans lequel la région constante d'immunoglobuline est la portion Fc de l'IgG1 humaine.

16. Corps ligand selon la revendication 14 ou 15, pour l'usage dans un procédé de traitement du corps humain ou animal, ou dans un procédé de diagnostic.
